# EUROPEAN PATENT APPLICATION

(11) **EP 3 828 895 A1**
(43) Date of publication of application: **02.06.2021**
(21) Application number: 19212153.1
(22) Date of filing: 28.11.2019
(51) Int. Cl.: G16H 50/00

(54) **SYSTEM AND METHOD FOR CONTROLLING A TIMING OF CONSECUTIVE BIOLOGICAL SAMPLES**

(71) Applicant: Radiometer Medical ApS, 2700 Brønshøj (DK)
(72) Inventor: Eriksen, Jakob Ohm, 2700 Brønshøj (DK)

(57) **Abstract**

System (10) for controlling a timing of consecutive biological samples (s0, s1) from a patient for a medical test (T), comprising:
a registration device (30), configured for determining a patient id (PID) indicating an identity of the patient, and a first sample time point (t0) indicating a time of obtaining a first biological sample (s0) from the patient at a first time point (t0);
a processing system (20), communicatively connected to the registration device (30), configured for
• receiving the patient id (PID) and the first sample time point (t0) from the registration device (30);
• determining a protocol rule (R) for the medical test (T) relating to timing requirements of obtaining biological samples (s0, s1) from the patient depending on the medical test (T); and
• determining a sample timing dependent on the protocol rule (R) and the first sample time point (t0), indicating when to obtain a second biological sample (s1) from the patient in compliance with the protocol rule (R).

## Description

### FIELD OF THE INVENTION

The present invention relates to controlling a timing of consecutive biological samples. In particular, the present invention relates to a system and method for controlling a timing of consecutive biological samples from a patient for a medical test.

### BACKGROUND OF THE INVENTION

The health status of a patient normally is not only determined based on the present symptoms but also on the result of different medical tests, in particular for different medical biomarkers. Depending on the result of such a test, the future treatment of a patient is determined, in particular, if a specific condition can be ruled out, ruled in or needs to be extendedly observed. For example, if a non-ST-segment elevation myocardial infarction, NSTEMI, is suspected, an electrocardiography, ECG, usually does not deliver reliable information. In such a case, a biological sample, in particular a blood sample, of the patient is tested for troponin, which is an indicating medical biomarker for an NSTEMI.

Normally, not only one test for the specific biomarker is run at a certain time, but at least a second measurement is executed in order to analyse the development of the biomarker over time.

In ruling in or out patients on clinical decision points a lack of control of exact sample timing of the biological samples may result in misdiagnosis, mistreatment of patients or even dismissal of false negative patients to home.

### SUMMARY OF THE INVENTION

Thus, there is a need for an improved system for controlling a timing of consecutive biological samples from a patient for a medical test.

The problem is solved by the subject matter according to the independent claims. The dependent claims, the following description and the drawings show preferred embodiments and further advantages of the invention.

Further on, it shall be noted that all embodiments of the present invention concerning the method might be carried out with the order of the steps as described explicitly hereinafter. Nevertheless, this has not to be the only and essential order of the steps of the method described herein. The herein presented method can be carried out with another order of the disclosed steps without departing from the respective method embodiments, unless explicitly mentioned to the contrary hereinafter.

Technical terms are implemented using their common sense. If a specific meaning is conveyed through certain terms, definitions of terms will be given in the following in the context of which the terms are used.

According to an aspect of the invention, a system for controlling a timing of consecutive biological samples from a patient for a medical test comprises a registration device, configured for determining a patient id indicating an identity of the patient, and a first sample time point indicating a time of obtaining a first biological sample from the patient at a first time point. The system further comprises a processing system, communicatively connected to the registration device, configured for receiving the patient id and the first sample time point from the registration device, determining a protocol rule for the medical test relating to timing requirements of obtaining biological samples from the patient depending on the medical test and determining a sample timing dependent on the protocol rule and the first sample time point, indicating when to obtain a second biological sample from the patient in compliance with the protocol rule.

The term "patient id", as used herein, comprises any form of identification of a patient. Preferably, the patient id comprises a code that can be interpreted by a processing system for identifying the patient. A combination of patient id and biological sample can thus be used to associate the biological sample to a patient that is further preferably already on file in the processing system.

Preferably, the registration device is configured as a portable or stationary device. The registration device is preferably configured for scanning a code relating to the patient or a biological sample. For example, the patient id is obtained or determined by the registration device by scanning a code on the medical record of the patient or scanning a code on a container intended for a biological sample of the specific patient. In the same way, the first sample time point is obtained or determined by the registration device by scanning a code on the container intended for the biological sample. Preferably, the container itself contains a container id that can be scanned by the registration device. Thus, an assignment of the patient to a specific biological sample in a container can be determined.

Preferably, the processing system comprises a computer or any suitable data processing system used for processing and/or storing data, in particular patient data and/or medical data. The processing system further preferably comprises at least one terminal for inputting or outputting data. Furthermore, the processing system is connected to additional devices, in particular devices for obtaining data, in particular patient data and/or medical data. An example for such devices is the aforementioned registration device. The processing system is preferably connected to the additional devices via a computer network, for example a local area network. The processing system thus manages the patient data and the associated medical data. Further preferably, concordance to sampling time requirements is relayed to a middleware central IT system for possible accreditation purpose.

The term "obtaining a biological sample", as used herein, preferably comprises drawing a blood sample from the patient, wherein the blood sample is analysed for a medical test.

Preferably, the sample time points, in particular the first sample time point, are determined directly at the patient, when obtaining the biological sample from the patient.

Preferably, for each medical test a protocol rule is predetermined. The protocol rule thereby contains conditions to be met to execute the medical test. In particular, the protocol rule contains timing requirements of obtaining the biological samples from the patient. In other words, the protocol rule determines when to obtain the biological samples from the patient in order to comply with the medical test. For example, a medical test for troponin in the biological sample determining NSTEMI has a protocol rule that a time between obtaining a first biological sample and a second biological sample is at least one hour. If the protocol rule is not followed, the test result will be flagged as non-compliant with protocol rule. The protocol rule thus comprises a predetermined range of sample timings in accordance with the medical test.

Preferably, the medical biomarkers comprise troponin I, TnI, troponin T, TnT, creatine kinase-muscle/brain, CKMB, myoglobin, Myo, N-terminal pro brain natriuretic peptid, NT-proBNP, procalcitonin, PCT, C-reactive protein, CRP, D-Dimer, β human chorionic gonadotropin βhCG.

The term "sample timing", as used herein, describes starting from the first sample time point, when a second biological sample needs to be taken from the patient to comply with the protocol rule of the intended medical test. Preferably, the sample timing contains a time point when the second biological sample needs to be obtained. Alternatively, the sample timing contains a time remaining for which the second biological sample must not be obtained in order to comply with the protocol rule.

Thus, the system provides an indication for a user or clinician, in particular a nurse, when to obtain the second biological sample from the patient in order to be in line with the protocol rule of the medical test. For example, the indication, when to obtain the second biological sample from the patient can be queried from the processing system and/or the registration device. Thus, the nurse has the option to check, if taking the second biological sample from the patient at this moment is too early and/or too late to be in line with the protocol rule can be reduced. Consequently, a result of the medical test is more reliable.

Thus, an improved system for controlling a timing of consecutive biological samples from a patient for a medical test is provided. Consequently, better guidance can be provided to the care takers in evaluating the patients' health status, specifically to rule in or rule out specific deceases.

In a preferred embodiment, the processing system is configured for providing a warning, if obtaining the second biological sample at a present time point does not comply with the protocol rule dependent on the determined sample timing.

The warning allows the user to be unaware of any additional information about the medical test or the timing of the obtaining the first biological sample. The user only has to check the provided warning, which for example indicates a positive or a negative feedback on taking the second biological sample. Thus, the probability that the nurse takes the second biological sample from the patient too early to be in line with the protocol rule can be reduced. Consequently, a result of the medical test is more reliable.

Thus, an improved system for controlling a timing of consecutive biological samples from a patient for a medical test is provided.

In a preferred embodiment, the registration device comprises a display, configured for displaying the warning provided by the processing system.

In order to provide a fast feedback from the processing system to the user, the registration device comprises a display for visual feedback of the warning. The warning preferably comprises an indication when to obtain the second biological sample. The indication preferably comprises a time to wait until obtaining the second biological sample from the patient is allowed in order to comply with the protocol rule. Further preferably, the indication comprises a time on a clock, at which the second biological sample should be obtained in order to comply with the protocol rule.

For example, the registration device comprises a request button. The request button is configured to be pressed to display the warning provided by the processing system.

Thus, an improved system for controlling a timing of consecutive biological samples from a patient for a medical test is provided.

In a preferred embodiment, the display is configured for displaying the warning in form of a colour code, in particular a traffic light code.

For a simple implementation of the warning, the registration device is configured to display a colour code. For example, the registration device comprises at least one lamp, in particular a LED, configured for displaying the colour code. For example, a green light indicates that the second biological sample can be obtained in order to comply with the protocol rule. A red light can indicate that the second biological sample must not yet be obtained to comply with the protocol rule.

The user thus just has to check the colour code for a predetermined colour in order to decide if the second biological sample should be obtained now.

Thus, an improved system for controlling a timing of consecutive biological samples from a patient for a medical test is provided.

In a preferred embodiment, the registration device is configured for determining the patient id and a second sample time point indicating a time of obtaining the second biological sample from the patient at a second time point different from the first sample time point, and the processing system is configured for receiving the patient id and the second sample time point.

Thus, the processing system is provided with the first time point and the second time point, each associated with a patient and a biological sample, via the patient id and preferably the container id.

Thus, the processing system is aware of the time the first biological sample and the consecutive second biological sample in fact have been obtained from the patient.

In a preferred embodiment, an analyser is configured for determining a value of a medical biomarker from a biological sample for a medical test, wherein a determined value of a medical biomarker of the second biological sample is marked as non-compliant, if the second sample time point does not comply with the determined sample timing.

For example, no warning, indicating if the second biological sample can be obtained to comply with the protocol rule, is present or the user ignores or misinterprets a provided warning, the system needs to make sure that a biological sample, which does not comply with the medical protocol, is not used for a clinical decision.

The processing system preferably determines a time difference between the first sample time point and the second sample time point. This time difference needs to be in line with the protocol rule in order to be reliable for the medical test.

If it is determined that the time difference does not comply with the protocol rule, for example, if the second biological sample has been obtained from the patient too early, the value of the medical biomarker of the second biological sample is marked as non-compliant and consequently not used for a clinical decision. Thus, it is prevented that the non-compliant value of the medical biomarker is believed to be compliant with the protocol rule and therefore affects a clinical decision. Alternatively, the value of the medical biomarker of the second biological sample is ignored or deleted to prevent the value of affecting a clinical decision.

Thus, it is prevented that a non reliable value of a medical biomarker of a biological sample is used for a clinical decision.

Thus, an improved system for controlling a timing of consecutive biological samples from a patient for a medical test is provided.

In a preferred embodiment, the sample timing comprises a minimal time difference between the first sample time point and a second sample time point indicating a time of obtaining the second biological sample from the patient at a second time point different from the first time point in order to comply with the protocol rule.

In general, the protocol rule of a medical test defines a minimal time difference between obtaining the first biological sample and obtaining the second biological sample. Taking the second biological sample later than an ideal time point of the protocol rule generally has a less significant negative impact on the reliability of a biological sample than taken the second biological sample too early.

In a preferred embodiment, the system comprises a database, containing a plurality of protocol rules each associated to a medical test, wherein determining the protocol rule for the medical test comprises selecting the protocol rule for the medical test from the plurality of protocol rules of the database dependent on the medical test.

The protocol rules are preferably predetermined dependent on the medical test they are used for. Storing all relevant protocol rules in a database allows for only determining the medical test to be performed and just selecting the associated protocol rule from the database. The medical test to be performed can be manually entered into the processing system, in particular via the registration device. However, the medical test to be performed can be determined automatically from a variety of input variables, for example the patient id, the container id and/or the biological sample. For example, the processing system was provided with the information, which patient is scheduled for which medical test. Alternatively, the processing system was provided with the information which medical test a specific container for the biological sample is scheduled for. Further alternatively, the type of medical biomarker analysed in the biological sample indicates, which medical test is performed on the patient. Preferably, a combination of manual input and automatic determination of the medical test to be performed is provided. In addition, preferably, at any time, a clinician is able to amend the protocol rule. For example, an additional third sample data point, for example at three hours after the first sample data point, can be manually entered into the processing system.

Further preferably, the present determined protocol rule can be read out of the processing system, in particular by a clinician, in order to have full control over the medical test. The system may already include a protocol, that defines a third, fourth or further sample to be drawn at e.g. three hours from the initial time point, e.g. after additional two hours from the second sample time point, under certain circumstances, e.g. if the delta value does not clearly indicate if the patient can be ruled out or in.

Thus, an improved system for controlling a timing of consecutive biological samples from a patient for a medical test is provided.

In a preferred embodiment, the first biological sample and the second biological sample are blood samples.

According to another aspect of the invention, a method for controlling a timing of consecutive biological samples from a patient for a medical test comprises the following steps. In a first step, a patient id indicating an identity of the patient is received and a first sample time point indicating a time of obtaining a first biological sample from the patient is received by a registration device. The patient id and the first sample time point from the registration device is received, by a processing system communicatively connected to the registration device. A protocol rule for the medical test relating to timing requirements of obtaining biological samples from the patient depending on the medical test is determined. A sample timing is determined dependent on the protocol rule and the first sample time point, indicating when to obtain a second biological sample from the patient in line with the protocol rule.

These and other features of the invention will become apparent from and elucidated with reference to the embodiments described hereinafter.

### BRIEF DESCRIPTION OF THE DRAWINGS

Exemplary embodiments of the invention will be described in the following drawings. The Figures are only schematic and not drawn to scale.
Fig. 1 schematically shows a diagram of the progression of a troponin concentration over time.
Fig. 2 schematically shows a diagram of the progression of a troponin concentration over time highlighting the sample timings.
Fig. 3 schematically shows a sample timing control method.
Fig. 4 schematically shows a system for controlling a timing of consecutive biological samples from a patient for a medical test providing a warning.
Fig. 5 schematically shows a system for controlling a timing of consecutive biological samples from a patient for a medical test providing a medical result.

### DETAILED DESCRIPTION OF EMBODIMENTS

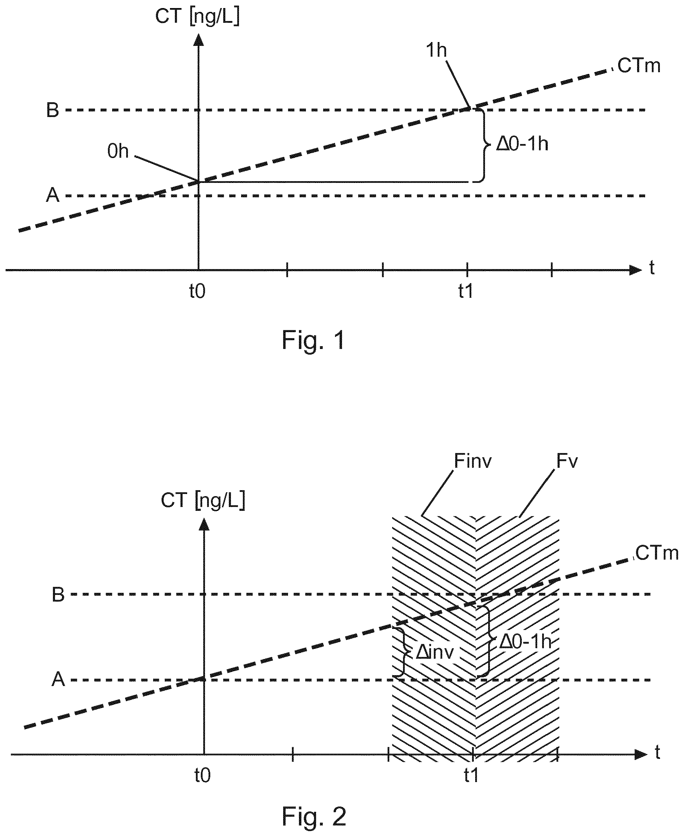

Fig. 1 schematically shows a diagram of a measured troponin development CTm, relating to the progression of a troponin concentration CT over time t. In this case, a non-ST-segment elevation myocardial infarction, NSTEMI, is suspected at a patient. Thus in a medical test, the blood of the patient is tested for troponin, which is an indicator for an NSTEMI. A so called 0 h/1 h rule-in and rule-out algorithm is performed. Basically, the troponin concentration CT of the blood of the patient is tested at least two times, in particular a first time and one hour later. In order to decide, if a NSTEMI is indicated by the troponin concentration CT, a predetermined protocol rule for this medical test is followed, in particular the ESC 2015 ACS-MI guideline accelerated protocols. Dependent on the measured troponin concentration CT and the development of the troponin concentration CT over time t, like the troponin delta Δ0-1h between the troponin concentration CT at a first time point t0 and the troponin concentration CT at a second time point t1, it is decided if an NSTEMI at the patient can be ruled-out, ruled-in or needs to be observed. Preferably, the second time point t1 is one hour later than the first time point t0.

Five thresholds are defined in the ESC 2015 ACS-MI guideline, a first threshold A, a second threshold B, a third threshold C (being the troponin delta Δ0-1h), a fourth threshold D (not shown) and a fifth threshold E (not shown). For example, according to the Architect technology, the first threshold A has a value of 2 ng/1, the second threshold B has a value of 5 ng/l, the third threshold C has a value of 2 ng/l, a fourth threshold D has a value of 52 ng/l and the fifth threshold E has a value of 6 ng/1.

A NSTEMI can be ruled-out, when a first sample dataset 0h, which is the troponin concentration CT at the first time point t0, is smaller than the first threshold A. Alternatively, a NSTEMI can be ruled-out, when the troponin concentration CT at the first time point t0 is smaller than the second threshold B and when the troponin delta Δ0-1h between the first sample dataset 0h and a second sample dataset 1h, which is the troponin concentration CT at the second time point t1.

A NSTEMI can be ruled-in, when the first sample dataset 0h is bigger or equal than the fourth threshold or when the troponin delta Δ0-1h is bigger or equal to the fifth threshold E.

A NSTEMI cannot be indicated but has to be observed in any other case.

As illustrated in the diagram of Fig. 1, the troponin concentration CT in the blood of the patient is measured at the first time point t0. This first sample dataset 0h has a value of about 3 ng/1. At the second time point t1, the troponin concentration CT in the blood of the patient is measured again. This second sample dataset 1h has a value of 4.9 ng/1. This leads to a troponin delta Δ0-1h of a 1.9 ng/1.

According to the predetermined protocol rules, a NSTEMI has to be ruled-out, since the troponin delta Δ0-1h has a value of less than the third threshold C. The patient can be send home, since the test came out negative.

Fig. 2 schematically shows a diagram of the progression of a measured troponin concentration CTm over time t highlighting the sample timings. Alternatively, the sample timing can also be used to prevent a wrong application of the tests. As illustrated in Fig. 2, at the second time point t1, the troponin delta Δ0-1h has a certain value. However, in order to compare this value with a rule set, like the aforementioned Architect technology, the timing of the second time point t1 needs to be as close to one hour after the first time point t0 as possible. Thereby, it is especially important that the timing of second time point t1 does exceed one hour after the first time point t0. In other words, in general, the second time point t1 normally can be too early in view of the first time point t0 to comply with the protocol rule, however it hardly can be too late in view of the first time point t0 to comply with the protocol rule.

Thus, as indicated in fig. 2, if the second blood sample has been taken too early, an invalid or non-compliant troponin delta Δinv is obtained, which should be ignored for a clinical decision. As can be seen in this example, the value of the invalid troponin delta Δinv is taken too early within an invalid time frame Finv to comply with the rule set. Such an invalid troponin delta Δinv would lead to a wrong diagnosis from the clinician as the troponin value in the blood of the patient did not have enough time to rise in view of the medical test to be performed. Thus, it has to be made sure that the second time sample t1 is within a valid time frame Fv.

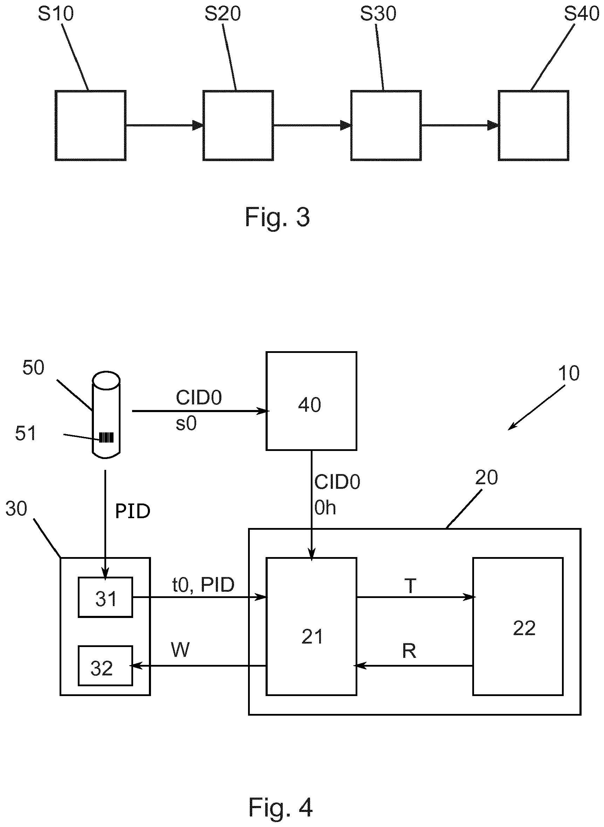

Fig. 3 schematically shows a method for controlling a timing of consecutive biological samples s0, s1 from a patient for a medical test T. In a first step S10, a patient id PID is determined by a registration device 30, indicating an identity of the patient and a first sample time point t0 indicating a time of obtaining a first biological sample s0 from the patient by a registration device 30. In a second step S20, the patient id PID and the first sample time point t0 is received from the registration device 30 by a processing system 20 communicatively connected to the registration device 30. In a third step S30, a protocol rule R is determined for the medical test T relating to timing requirements of obtaining biological samples s0, s1 from the patient depending on the medical test T. In a fourth step S40, a sample timing is determined dependent on the protocol rule R and the first sample time point t0, indicating when to obtain a second biological sample s1 from the patient in line with the protocol rule R.

Fig. 4 schematically shows a system 10 for controlling a timing of consecutive biological samples s0, s1 from a patient for a medical test.

The system comprises a processing system 20, a registration device 30 and an analyzer device 40. In this case, a patient should be tested for NSTEMI. Thus, a troponin concentration of the blood of the patient should be measured. Thus the biological samples s0, s1 to be obtained from the patient are blood samples s0, s1.

A clinician takes a first blood sample s0 from the patient. The first blood sample s0 is drawn from the patient into a container 50. The first blood sample s0 and the container 50 need to be associated to the treated patient. Therefore, the clinician or any kind of care taker either enters a patient id PID associated with the patient into the registration device 30. Alternatively, as shown in this example, the container 50 comprises a barcode 51, which comprises information about the identification of the patient. For example, in a previous step, the barcode 51 of the container 50 was assigned to a specific patient. In this case, the registration device 30 comprises a registration unit 31, in particular a scanner, configured for scanning the barcode 51 of the container 50. Thus, the registration device 30 obtains the patient id PID associated with the first blood sample s0. Alternatively, the patient id PID is obtained by the registration device 30 by scanning a barcode 51 placed on the bed of the patient. Additionally, for compliance reasons, preferably a nurse id is obtained by the registration device 30, in particular by entering an identification of the nurse, for example an identification code, into the registration device 30 before the registration device 30 can be used. In addition, the registration device 30 is configured to determine a first sample time point t0 corresponding to the time point at which the barcode 51 has been scanned by the registration device 30. In other words, the registration device 30 is configured to sample the time, for example of an internal clock of the registration device, at the time point at which the barcode 51 has been scanned by the registration device 30. The first sample time point t0 thus represents the time point at which the container 50 has been scanned, which should be very close to the time point the first blood sample s0 has been drawn from the patient.

In addition the patient id PID, the barcode 51 comprises information about the container 50 itself, stored in a first container id CID0. The first container id CID0 identifies the container 50 containing the first blood sample s0. Thus, when the first blood sample s0 is provided to the analyzer device 40, the analyzer device 40 can scan the barcode 51 and read out the first container id CID0 and associate the first blood sample s0 to a specific container 50.

Thus, the registration device 30 is configured for obtaining the patient id PID, the first container id CID0, the nurse id and the first sample time point t0. In other words, the registration device 30 is configured for obtaining any necessary information about the blood sample s0 and its container, the patient and the clinician or nurse handling the registration device 30 for the analysis of the medical test.

Thus, the system 10 can associate the container 50 with the first blood sample s0 via the first container id CID0. In addition, the system 10 can associate the first blood sample s0 with the patient via the patient id PID. Lastly, the system 10 can associate the first sample time point t0 with the patient and the first blood sample s0. Thus, it is known, which blood sample s0 is from which patient and stored in which container 50 and obtained at which time t0.

The analyzer device 40 is configured to analyse the provided first blood sample s0, in this case for the biomarker troponin. Thus, the analyzer device 40 determines from the first blood sample s0 a first sample dataset 0h, representing a concentration of troponin in the first blood sample s0 at the first sample time point t0. The determined first sample dataset 0h is provided to the processing system together with the associated first container id CID0.

The processing system 20 uses the provided information to determine, when it is the best time point to obtain a second blood sample s1 from the patient. In this case, the patient is tested for NSTEMI. Thus, the troponin concentration of the blood of the patient has to be tested at two different time points, a first sample time point t0 and a second sample time point t1, at least 1 hour apart from another. In other words, depending on the development of the troponin concentration of the blood of the patient, drawn at two different time points, it can be clinically estimated if an NSTEMI is present at the tested patient. The time difference between the first sample time point t0 and the second sample time point t1 necessary for a reliable estimation is defined in a protocol rule. The protocol rule depends on the medical test applied to the patient.

Thus, the processing system 20 comprises a processing unit 21 and a database 22. The database 22 contains a plurality of protocol rule R, each associated with a medical test T.

The processing unit 21 requests a relevant protocol rule R from the database 22. Thus, the processing unit 21 needs to be aware of the medical test T, applied to the patient. A plurality of possibilities are thinkable. The processing unit 21 may determine the medical test T based on the provided first container id CID0, when the processing system 21 previously was provided with an association between container ids and medical tests. In other words, if the processing system 21 got the information, which container 50 is used for which medical test T, the processing system 21 is able to determine the medical test T from the first container id CID0. Alternatively, the processing system 21 was provided with the information which patient is scheduled for which medical test T. Thus, the processing system 21 is able to determine the medical test T from the patient id PID. Alternatively, the processing system 21 is configured for determining the medical test T based on the provided first sample dataset 0h. In other words, the processing system 21 may be able to determine the medical test T based on the type of provided biomarker. In this case, the processing system 21 may be able to determine that troponin only is used for a NSTEMI test. Alternatively, the medical test T is just inputted into the processing system, in particular via an input interface, for example a keypad.

In this case, the protocol rule R determines that the first sample time point t0 and the second sample time point t1 need to be apart from another at least 1 hour to comply with the protocol rule R. Thus, the processing unit 21 determines a sample timing dependent on the protocol rule R and the first sample time point t0, indicating when to obtain a second blood sample s1 from the patient in compliance with the protocol rule R. The sample timing in this case comprises an earliest time point at which the second blood sample s1 should be obtained from the patient.

Based on the sample timing, the processing unit 21 determines a warning W, which is provided back to the registration device 30. The warning W is then displayed on a display 32 of the registration device 30. The clinician thus can check the registration device 30 before taking the second blood sample s1 from the patient in order to be sure to comply with the protocol rule R. For example, the warning W comprises a time left before taking the second blood sample s1. The clinician thus is provided with valuable information about compliance of the blood samples s0, s1 with the protocol rule R. Thus, the reliability of the medical test T can be improved.

The described IT infrastructure between the registration device 30, the processing system 20 and the analyzer device 40 comprises interconnecting a plurality of clinical devices and/or communication devices, like analyzers, middle ware systems, phones PDAs and scanners. The analyzer device 40 for example is a mobile analyzing device, like for example an AQT-analyzer, or a stationary analyzer in a laboratory.

In case of cut-off scenarios, in other words, when the prediction P of an NSTEMI being present at a patient is a close decision, the improved reliability of the provided system leads to an improved decision process. In other words, due to the improved accuracy of the prediction P, in less cases, false positive predictions and false negative predictions. In a false positive prediction, the patient will be unnecessarily hold in the clinic for further tests. In a false negative prediction, the patient will be send home, although further medical care might be necessary.

Thus, the provided system decreases the amount of false positive predictions and false negative predictions.

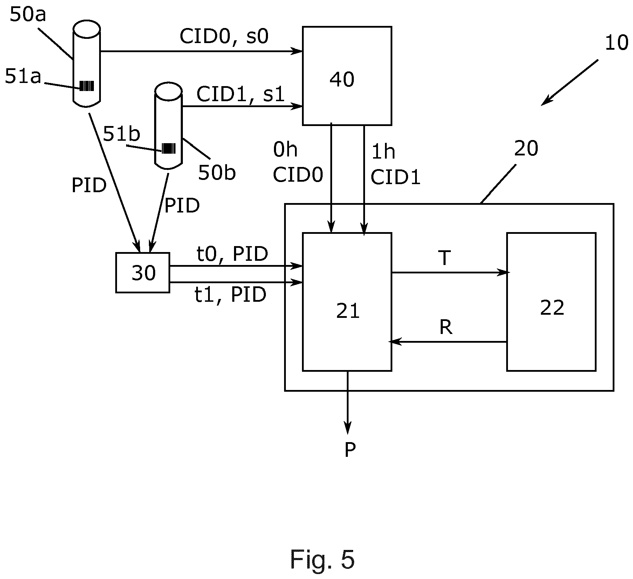

Fig. 5 also schematically shows a system 10 for controlling a timing of consecutive biological samples s0, s1 from a patient for a medical test.

In contrast to the situation described in fig. 4, in this case, the second blood sample s1 has already been obtained from the patient. In line with the description above, the registration device 30 scans a first barcode 51a of a first container 50a with a first blood sample s0 at a first sample time point t0. At a second sample time point t1, the registration device 30 scans a second barcode 51b of a second container 50b with a second blood sample s1. Thus, the first barcode 51a contains a first container id CID0 and the second barcode 51b contains a second container id CID1.

The analyzer device 40 is provided with the respective first blood sample s0 and second blood sample s1 as well as the associated first container id CID0 and the second container id CID1. Based on that the analyzer device 40 determines a first sample dataset 0h and a second sample dataset 1h.

In line with the description of fig. 4, the processing unit 21 is provided with the patient id PID, the respective container ids CID0, CID1, the respective sample datasets 0h, 1h and the respective sample time points t0, t1. The processing unit 21 also is provided with the relevant protocol rule R from the database 22 after determining the relevant medical test T.

However, in this case, no warning W is provided to the registration device 30, since already both blood samples have been analysed. The processing unit 21 rather determines a prediction P, which is the result of the medical test T based on the first sample dataset 0h and the second sample dataset 1h. Before determining the prediction P however, the processing unit 21 checks, if the first sample time point t0 and the second sample time point t1 comply with the provided protocol rule R. if the sample time points t0, t1 do not comply with the protocol rule R, the processing unit 21 flags the provided sample datasets 0h, 1h. In particular, the processing unit marks the second sample dataset 1h as non-compliant, if it is determined that the second sample time point t1 does not comply with the protocol rule R. The clinician can then decide if he still uses the second sample dataset 1h as basis for a clinical decision.

Thus, when a prediction P for the result of the medical test T is provided, the clinician receiving the prediction P can be sure that the blood samples s0, s1 were in line with the protocol rule R and thus relatively reliable. The clinician thus can make an improved clinical decision based on the provided prediction.

### REFERENCE SIGNS

- 0h: first sample dataset
- 1h: second sample dataset
- t0: first sample time point
- t1: second sample time point
- A: first threshold
- B: second threshold
- CT: troponin concentration
- CTm: measured troponin development
- Δ0-1h: troponin delta
- Δinv: invalid troponin delta
- Fv: valid time frame
- Finv: invalid time frame
- 10: system
- 20: processing system
- 21: processing unit
- 22: database
- 30: registration device
- 31: registration unit
- 32: display
- 40: analyzer device
- 50: container
- 51: barcode
- s0: first biological sample (first blood sample)
- s1: second biological sample (second blood sample)
- PID: patient id
- CID0: first container id
- CID1: second container id
- R: protocol rule
- T: medical test
- W: warning
- P: prediction
- S10: determining a patient id and a first sample time point
- S20: receiving the patient id and the first sample time point
- S30: determining a protocol rule
- S40: determining a sample timing

## Claims

1. System (10) for controlling a timing of consecutive biological samples (s0, s1) from a patient for a medical test (T), comprising:
a registration device (30), configured for determining a patient id (PID) indicating an identity of the patient, and a first sample time point (t0) indicating a time of obtaining a first biological sample (s0) from the patient at a first time point (t0);
a processing system (20), communicatively connected to the registration device (30), configured for
• receiving the patient id (PID) and the first sample time point (t0) from the registration device (30);
• determining a protocol rule (R) for the medical test (T) relating to timing requirements of obtaining biological samples (s0, s1) from the patient depending on the medical test (T); and
• determining a sample timing dependent on the protocol rule (R) and the first sample time point (t0), indicating when to obtain a second biological sample (s1) from the patient in compliance with the protocol rule (R).

2. System of claim 1, wherein the processing system is configured for
providing a warning (W), if obtaining the second biological sample (s2) at a present time point does not comply with the protocol rule (R) dependent on the determined sample timing.

3. System of claim 2, wherein
the registration device (30) comprises a display (32), configured for displaying the warning (W) provided by the processing system (20).

4. System of any claim 3, wherein
the display (32) is configured for displaying the warning (W) in form of a colour code, in particular a traffic light code.

5. System of any of the preceding claims, wherein
the registration device (30) is configured for determining the patient id (PID) and a second sample time point (t1) indicating a time of obtaining the second biological sample (s1) from the patient at a second time point (t1) different from the first sample time point (t0); and
the processing system (20) is configured for receiving the patient id (PID) and the second sample time point (t1).

6. System of claim 5, comprising:
an analyser (40) configured for determining a value of a medical biomarker from a biological sample (s0, s1) for a medical test (T);
wherein a determined value of a medical biomarker of the second biological sample (s1) is marked as non-compliant, if the second sample time point (t1) of the second biological sample (s1) does not comply with the determined sample timing.

7. System of any of the preceding claims, wherein
the sample timing comprises a minimal time difference between the first sample time point (t0) and a second sample time point (t1) indicating a time of obtaining the second biological sample (s1) from the patient at a second sample time point (t1) different from the first sample time point (t0) in order to comply with the protocol rule (R).

8. System of any of the preceding claims, comprising:
a database (22), containing a plurality of protocol rules (R) each associated to a medical test (T); wherein determining the protocol rule (R) for the medical test (T) comprises:
selecting the protocol rule (R) for the medical test (T) from the plurality of protocol rules (R) of the database (22) dependent on the medical test (T).

9. System of any of the preceding claims, wherein
the first biological sample (s0) and the second biological sample (s1) are blood samples.

10. Method for controlling a timing of consecutive biological samples (s0, s1) from a patient for a medical test (T), comprising the steps:
determining (S10) a patient id (PID) indicating an identity of the patient and a first sample time point (t0) indicating a time of obtaining a first biological sample (s0) from the patient by a registration device (30);
receiving (S20) the patient id (PID) and the first sample time point (t0) from the registration device (30), by a processing system (20) communicatively connected to the registration device (30);
determining (S30) a protocol rule (R) for the medical test (T) relating to timing requirements of obtaining biological samples (s0, s1) from the patient depending on the medical test (T); and
determining (S40) a sample timing dependent on the protocol rule (R) and the first sample time point (t0), indicating when to obtain a second biological sample (s1) from the patient in line with the protocol rule (R).
